# EUROPEAN PATENT APPLICATION

(11) **EP 3 868 394 A1**
(43) Date of publication of application: **25.08.2021**
(21) Application number: 20158493.5
(22) Date of filing: 20.02.2020
(51) Int. Cl.: A61K 38/22, A61K 51/08, C07K 7/02

(54) **MONO- AND MULTI-TRIAZOLOMINIGASTRINS FOR TARGETING OF CCK2R-POSITIVE NEOPLASMS**

(71) Applicant: PAUL SCHERRER INSTITUT, 5232 Villigen PSI (CH)
(72) Inventor: GROB, Nathalie, 8057 Zürich (CH); MINDT, Thomas L., 1180 Wien (AT); BEHE, Martin, 4460 Gelterkinden (CH); SCHIBLI, Roger, 5400 Baden (CH); VALVERDE, Ibai, 21160 Marsannay la Côte (FR)
(74) Representative: Fischer, Michael

(57) **Abstract**

It is the objective of the present invention to provide minigastrin derivates that have a high receptor specific cell internalization, excellent IC₅₀ values towards the CCK2R and sufficient plasma stability.

This objective is achieved according to the present invention by a minigastrin derivate, in particular mono- and multi-triazolominigastrins for targeting of CCK2R positive neoplasms, having the formula:
X-Z-Ψ[Tz]-Ala-Ψ[Tz]-Tyr-Ψ[Tz]-Gly-Trp-Nle-Asp-Phe-NH₂ (Y), or
X-Z-DGlu-Ψ[Tz]-Ala-Tyr-Ψ[Tz]-Gly-Trp-Nle-Asp-Phe-NH₂ (Y), or
X-Z-DGlu-Ala-Tyr-Ψ[Tz]-Gly-Trp-Nle-Asp-Phe-NH₂ (Y),
while Ψ[Tz] stands for a 1,4-disubstituted or a 1,5-disubstituted 1,2,3-triazole, X stands for a chemical group attached to the peptide for the purpose of diagnostic and/or therapeutic intervention at CCK2R-relevant diseases, Y stands for C-terminal modifications of the peptide, such as amide, primary and secondary amides, free carboxylic acids and carboxylic ester derivatives including but not limited to amides and esters derived from linear or branched alkyl-, alkenyl-, alkynyl- aromatic-, and heterocyclic alcohols, and Z stands for a linker or DGlu* wherein DGlu* stands for a chain of DGlu having 1 to 6 repetitions (-DGlu- to -DGlu-DGlu-DGlu-DGlu-DGlu-DGlu-).

These minigastrin derivates have specifically a high receptor specific cell internalization, excellent IC₅₀ values towards the CCK2R and sufficient plasma stability.

## Description

The present invention relates to mono- and multi-triazolominigastrins for targeting of CCK2R-positive neoplasms.

G protein-coupled receptors (GPCRs) are used as target proteins for radiolabeled peptides since the early 90's. The somatostatin receptor was the prototype for radio-nuclide imaging and therapy with peptides (Lit) resulting in a clinical first-line therapy for neuroendocrine tumors with ⁹⁰Y and ¹⁷⁷Lu labeled derivatives of octreotide (Lit). Several radiolabeled peptides were tested for the possibility to target overexpressed GPCRs on tumors including gastrin-releasing peptide analogs (GRP), glucagon-like peptide 1 analogs (GLP-1), neurotensin analogs (NT) or neuropeptide Y analogs (NPY) (Mäcke, Reubi J Nucl Med 2008; 49:1735-1738). An additional very interesting target is the cholecystokinin-2 receptor (CCK2R). This receptor is mainly expressed on medullary thyroid carcinomas (MTC), small cell lung cancers (SCLC), and stromal ovarial tumors (Reubi, Int J Cancer. 1996 and Reubi, Cancer Res. 1997). Radiolabeled gastrin analogs are good candidates for targeted imaging and therapy. It was shown that ¹¹¹In-labeled gastrin analogs are superior for detecting MTC compared to [¹¹¹In]In-OctreoScan and give additional information on neuroendocrine tumors particularly if they are negative in somatostatin receptor scintigraphy (Endocr Relat Cancer. 2006 Dec;13(4):1203-11.; Eur J Nucl Med Mol Imaging. 2006 Nov;33(11):1273-9).

However, due to the high kidney uptake the radiolabeled peptides could not be used for therapy. The high kidney uptake is correlated with the six negatively charged glutamic acids. 12 gastrin-related compounds were designed, synthesized and compared as ¹¹¹In-labeled compounds. The best compounds with respect to a high tumor-to-kidney ratio are the minigastrins with six D-glutamic acids or six glutamines. These compounds still possess a methionine which can be oxidized easily. This is a disadvantage for clinical application since the receptor affinity is dramatically decreased after oxidation of methionine, and the production under GMP conditions may be hampered.

A high potential for a significant improvement of the therapy and the image generation with patients having metastasized MTC, SCLC, and further CCK2R-positive tumors has a specific labeling of the tumor cells with radiolabeled analogs of gastrin. Basis for this finding is the over-expression of the respective target (CCK2R) in 92% of the investigated MTC [Reubi 1997]. Furthermore, the same working group identified the same over-expression of the target receptor (CCK2R) in 57% of small cell lung cancers, 65% of astrocytomas and 100% of stromal ovarian tumors.

First therapy studies (phase 0 study) had been executed including eight patients with advanced metastasized MTC. A partial remission was achieved for two patients, four patients showed a stabilization of the formerly progressive course of the disease after a therapy with a ⁹⁰Y-labeled analog of minigastrin. This study had to be stopped due to the nephrotoxicity of the therapy resulting from accumulation of the radioactivity in the kidneys.

With support of the European COST initiative (European Cooperation in Science and Technology), in the meantime a plurality of significantly improved radiolabeled analogs of gastrin have been synthesized by various working groups and have been investigated for their characteristics in vitro an in vivo. As compared to the former gastrin analog, these newer substances possess a significantly higher tumor-to-kidney ratio with respect to the absorption in human tissue [Laverman 2011, Polenc-Peitl 2011, Ocak 2011, Fani 2012]. Currently, out of these newer analogsof gastrin, [¹⁷⁷Lu]Lu-PP-F11 (DOTA-DGlu-DGlu-DGlu-DGlu-DGlu-DGlu-Ala-Tyr-Gly-Trp-Met-Asp-Phe-NH₂, hereinafter called PP-F11) exhibited promising properties for future radio nuclide therapy due to its high favorable accumulation in the tumor accompanied by a low accumulation in the kidneys.

PP-F11 has been further improved according to the international patent application WO 2015/067473 A1 wherein the minigastrin analog PP-F11 having the formula: X-DGlu-DGlu-DGlu-DGlu-DGlu-DGlu-Ala-Tyr-Gly-Trp-Y-Asp-Phe-NH2, wherein Y stands for an amino acid replacing methionine and X stands for a chemical group attached to the peptide for the purpose of diagnostic and/or therapeutic intervention at CCK2R relevant diseases. In particular, very suitable compounds with respect to a high tumor-to-kidney ratio are minigastrin derivates with six D-glutamic acids or six glutamines. These compounds still possess a methionine which can be oxidized easily. This is a disadvantage for clinical application under GMP conditions due to the side products which may occur during the radiolabeling procedure.

Therefore, the substitution of methionine with a non-oxidizable isosteric amino acid avoids the oxidation during storage and production which could lead to lower affinity compound. In a preferred embodiment according to WO 2015/067473 A1, methionine is replaced by norleucine. This so-called PP-F11N minigastrin exhibits excellent tumor-to-kidney ratio and is therefore a highly promising candidate for clinical applications.

Further, WO 2019/057445 A1 discloses minigastrin derivates having the formula:

X-Z-Ala-Tyr-Gly-Trp-Met-Asp-Phe-NH₂ (Y),

wherein at least one of the connecting or terminal amide bonds between, before, or after the amino acids of the sequence Z, Ala, Tyr, Gly, Trp, Met, Asp, Phe and NH₂ or Y (C-terminal) is replaced by a 1,4-disubstituted or a 1,5-disubstituted 1,2,3-triazole, while X stands for a chemical group attached to the peptide for the purpose of diagnostic and/or therapeutic intervention at CCK2R-relevant diseases, Y stands for C-terminal modifications of the peptide, such as amide, primary and secondary amides, free carboxylic acids and carboxylic ester derivatives including but not limited to amides and esters derived from linear or branched alkyl-,alkenyl-, alkynyl- aromatic-, and heterocyclic alcohols, and Z stands for a linker or DGlu* wherein DGlu* stands for a chain of DGlu having 1 to 6 repetitions (-DGlu-to -DGlu-DGlu-DGlu-DGlu-DGlu-DGlu-).

These minigastrin derivates have a high receptor-specific cell internalization, excellent IC₅₀ values towards the CCK2R, and sufficient plasma stability. Preferably, methionine is replaced by norleucine or another amino acid which conserve the affinity to the receptor, preferably to give minigastrin derivate [Nle¹⁵]-MG11 having one DGlu only.

Nonetheless, there is still a need to provide further suitable minigastrin derivates in order to enlarge the variety of compounds that are potentially available in the desired afore-mentioned pharmaceutical applications. It is therefore the objective of the present invention to provide further minigastrin derivates that have a high receptor-specific cell internalization, excellent IC₅₀ values towards the CCK2R, and sufficient plasma stability.

This objective is achieved according to the present invention by a minigastrin derivate, in particular mono- and multi-triazolominigastrins for targeting of CCK2R-positive neoplasms, having the formula:

X-Z-Ψ[Tz]-Ala-Ψ[Tz]-Tyr-Ψ[Tz]-Gly-Trp-Nle-Asp-Phe-NH₂ (Y),

or

X-Z-DGlu-Ψ[Tz]-Ala-Tyr-Ψ[Tz]-Gly-Trp-Nle-Asp-Phe-NH₂ (Y),

or

X-Z-DGlu-Ala-Tyr-Ψ[Tz]-Gly-Trp-Nle-Asp-Phe-NH₂ (Y),

while Ψ[Tz] stands for a 1,4-disubstituted or a 1,5-disubstituted 1,2,3-triazole, X stands for a chemical group attached to the peptide for the purpose of diagnostic and/or therapeutic intervention at CCK2R-relevant diseases, Y stands for C-terminal modifications of the peptide, such as amide, primary and secondary amides, free carboxylic acids and carboxylic ester derivatives including but not limited to amides and esters derived from linear or branched alkyl-,alkenyl-, alkynyl- aromatic-, and heterocyclic alcohols, and Z stands for a linker or DGlu* wherein DGlu* stands for a chain of DGlu having 1 to 6 repetitions (-DGlu- to -DGlu-DGlu-DGlu-DGlu-DGlu-DGlu-).

In particular, these minigastrin derivates have a high receptor-specific cell internalization, excellent IC₅₀ values towards the CCK2R, and sufficient plasma stability.

With respect to peptide receptor radionuclide therapy (PRRT), X may stand for a radionuclide including the attachment group like a chelator for radiometals, such as ¹⁷⁷Lu or ⁹⁰Y or ¹¹¹In, or a prosthetic group for non-metals like ¹⁸F , ¹¹C, radioiodines, or a functional group suitable for pre-targeting approaches. In order to improve the medical imaging, the X may stand for an optically active chemical compound, such Alexa Fluor® 647, IRDye 680RD, DY-700 or any other photoactive substance. For optical therapeutic applications, it may be a photosensitizer like Photofrin, Forscam or Photochlor. For both applications the active chemical compound may be an optical active nanoparticle. In order to support the chemotherapeutic intervention, X may stand for a chemotherapeutic active compound, such as gemcitabine, doxorubicin or cyclophosphamide. X may also stand for a combination of an imaging agent (dye, radionuclide) and a therapeutic entity (cytotoxic compounds, radionuclide). The delivery of the described agents may be done by a nanoparticle or liposome as X whereas they are loaded with chemotherapeutic agents.

Z may also present a linker or spacer unit that covalently connects the peptide with the imaging probe or therapeutic while keeping them at distance in order to avoid potential interference with the biological properties of the peptide. Linker moieties may include but are not limited to linear or branched alkyl chains of 1-20 carbon length containing but not limited to 1-10 heteroatoms (including O, S, N, P), all carbon or heterocyclic aromatic moieties such as phenyl, naphthalene, triazoles, thiophenes, furans etc., and unsaturated C-C or C-heteroatom (O, N, S, P) bonds either in the main linker chain or in one or several sidechains.

Preferably, X stands for a chelator wherein one chelator for radiometals can be DOTA (1,4,7,10-tetraazacyclododecane-1,4,7,10-tetraacetic acid) and/or the radionuclide is selected from the group consisting of ¹⁷⁷Lu, ⁶⁷Ga/⁶⁸Ga, ⁹⁰Y, and ¹¹¹In. Other radionuclides (^{99m}Tc, ⁶⁴Cu/⁶⁷Cu, ²⁵⁵Ac, ²¹³Bi, ²¹²Pb, ²²⁷Th) and other suitable chelators are known to those skilled in the art, such as MAG3, HYNIC, NOTA, NODAGA, DOTAGA, CHX-A"-DTPA, DFO, TCMC, HEHA, sarcophagines, cross-bridged versions of cyclam, and cylcen chelators.

Particularly suitable minigastrin derivates have the following formula:
DOTA-DGlu-Ψ[Tz]-Ala-Ψ[Tz]-Tyr-Ψ[Tz]-Gly-Trp-Nle-Asp-Phe-NH₂ or
DOTA-(DGlu)₅-DGlu-Ψ[Tz]-Ala-Tyr-Ψ[Tz]-Gly-Trp-Nle-Asp-Phe-NH₂.

In a further preferred embodiment of the present invention, X may also stand for a nanoparticle or a liposome which have a diagnostic function (e.g. optical active or MRI contrast agent) or which have therapeutic function by themselves or are loaded with an active compound.

With respect to the use of the inventive minigastrin derivate, a diagnostic intervention at CCK2R-relevant diseases and/or a therapeutic intervention at CCK2R-relevant diseases is/are foreseen.

Preferred embodiments of the present invention are hereinafter described in more detail with respect to the attached drawings which depict in:
- Figure 1: the total cell internalization of ¹⁷⁷Lu-labeled [Nle¹⁵]-MG11, PP-F11N, and a number of mono- and multi-triazolominigastrins;
- Figure 2: the half maximal inhibitory concentration IC₅₀ of ^{nat}Lu-labeled [Nle¹⁵]-MG11, PP-F11N, and a number of mono- and multi-triazolominigastrins;
- Figure 3: the plasma stability of ¹⁷⁷Lu-labeled [Nle¹⁵]-MG11, PP-F11N, and a number of mono- and multi-triazolominigastrins;

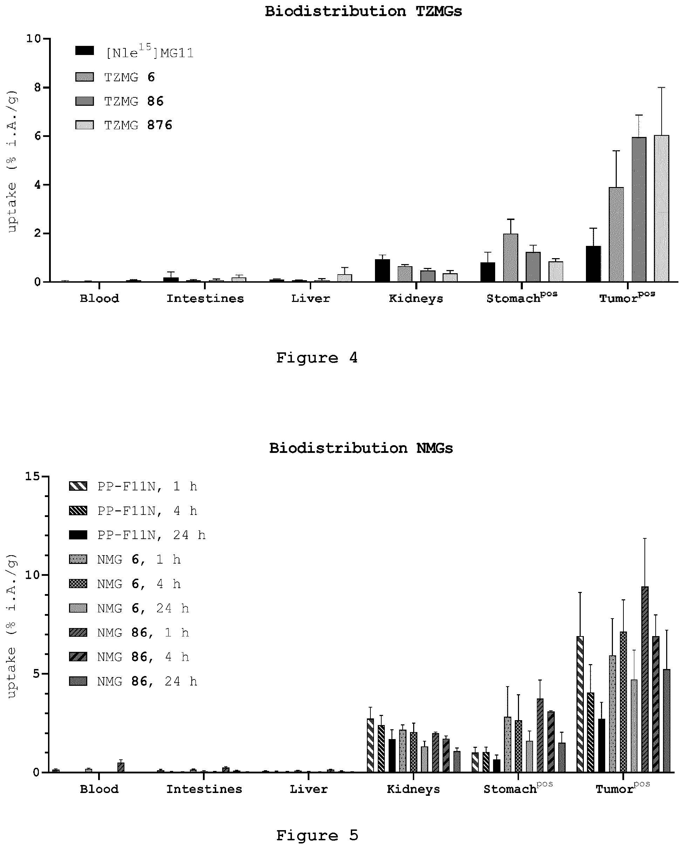

- Figure 4: the biodistribution studies of ¹⁷⁷Lu-labeled [Nle¹⁵]MG11 and TZMGs in mice with CCK2R-positive tumor xenografts at 4 hours post injection; and
- Figure 5: the biodistribution studies of ¹⁷⁷Lu-labeled PP-F11N and NMGs in mice with CCK2R-positive tumor xenografts at 1, 4, and 24 h post injection.

[Nle¹⁵]-MG11 is a truncated analog of minigastrin, a regulatory peptide with high affinity and specificity towards the CCK2R, which is overexpressed in various types of cancer. The N-terminal conjugation of 1,4,7,10-tetraazacyclododecane-1,4,7,10-tetraacetic acid (DOTA) allows for radiolabeling of the peptide with metallic radionuclides (e.g., ¹⁷⁷Lu) and subsequent use for *in vivo* tumor imaging and peptide receptor radionuclide therapy.

A drawback of using MG11 as a tumor-targeting vector is its low tumor uptake due to fast enzymatic degradation, which results in a biological half-life of only a few minutes. The systematic replacement of single amide bonds in the peptide sequence by stable 1,4-disubstituted or 1,5-disubstituted 1,2,3-triazoles, termed a triazole-scan, leads to an improved proteolytic stability and tumor-targeting properties. To prove the general utility of this methodology, a triazole-scan was performed with DOTA[Nle¹⁵]-MG11 by a solid-phase approach employing a combination of SPPS and the Copper(I)-catalyzed azide-alkyne cycloaddition (CuAAC) for 1,4-disubstituted 1,2,3-triazoles. The introduction of 1,5-disubstituted 1,2,3-triazoles is analogous but employing the Ruthenium-catalyzed azide-alkyne cycloaddition (RuAAC). In particular, TZMG 876 and NMG 86 (see table 1 below) have been investigated in more detail.

The following examples discuss the synthesis of radiolabeled triazolopeptidomimetics and the evaluation of their physiochemical properties. Receptor affinities (IC₅₀), tumor cell internalization rates, and plasma stabilities of the peptide conjugates were investigated *in vitro.* First results of *in vivo* studies with xenografted mice have been achieved. The ultimate goal of this project is to identify a number of minigastrin derivates different from DOTA[Nle¹⁵]-MG11 or PP-F11N with maintained or improved biological activity or superior resistance to enzymatic degradation leading to improved uptake in tumors.

The building blocks for the CuAAC, RuAAC, and triazolopeptidomimetics have been prepared by adapted procedures reported in literature by Valdere et al., Mascarin et al., and WO 2019/057445 A1.

### General Procedures A: Synthesis of Weinreb Amides

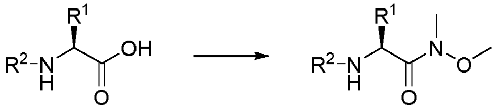

**R¹ = amino acid specific side chain**
**R² = Boc/Fmoc**

The corresponding Fmoc- or Boc- protected amino acid (1 equiv.) was dissolved in CH₂Cl₂ (0.1 M), and DIPEA (2.5 equiv.), and BOP (1 equiv.) were added. The solution was stirred for 15 min, N,O-dimethylhydroxylamine (1.2 equiv.) was added and the reaction was stirred for 12-14 h at rt.

The solution was diluted with CH₂Cl₂, washed with 0.1 M HCl (3x), saturated aq. NaHCO₃ (3x), and water (3x). The organic phase was dried over MgSO₄, filtered, and the solvent was removed under reduced pressure. The desired Weinreb amide was isolated by flash chromatography on silica gel.

### General Procedures B: Synthesis of α-Amino Alcohols

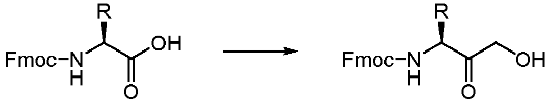

### R = amino acid specific side chain

For amino acids with a protected carboxylic acid in their side chain, the corresponding Fmoc-protected amino acid (1 equiv.) was dissolved in anhydrous THF (0.2 M) under Argon and cooled to 0 °C (ice bath). N-Methylmorpholine (1.1 equiv.) and isobutyl chloroformate (1.05 equiv.) were added and the reaction was stirred for 15 min at 0 °C. Completion of the reaction was monitored by TLC. The white suspension was added dropwise to a precooled suspension of NaBH₄ (2 equiv.) in THF/MeOH (3:1, or pure THF) at -78 °C (dry ice, acetone) and stirred for 20 min. Upon completion of the reduction, residual hydrides were quenched by aq. 10% acetic acid, and the solution was concentrated under reduced pressure. The residue was extracted with EtOAc (3x), and the organic layer was washed with aq. sat. NaHCO₃ (2x), and water (1x). The organic layer was dried over Na₂SO₄, filtered and the solvent was removed under reduced pressure. The desired α-amino alcohol was isolated by flash chromatography on silica gel.

### General Procedures C: Synthesis of α-Amino Alkynes

### C.1 Synthesis of α-Amino Alkynes from Boc-protected Weinreb amides

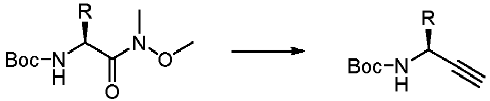

R = amino acid specific side chain

The corresponding Weinreb amide (1 equiv.) was placed in a flame dried flask under argon and dissolved in anhydrous CH₂Cl₂ (0.1 M). The solution was cooled to -78 °C (dry ice/acetone bath) and 1 M DIBAL-H in toluene was added dropwise (3 equiv.). After 1 h of stirring, the reaction was checked for completion by TLC. If the reaction was not finished, 1 M DIBAL-H in toluene (1 equiv.) was added, and the reaction was stirred again for 1 h at -78 °C. After consumption of the starting material, the excess hydride was quenched by slow addition of anhydrous MeOH and the reaction was allowed to warm to 0 °C (ice/water bath). K₂CO₃ (3 equiv.), dimethyl-(1-diazo-2-oxopropyl)phosphonate (2 equiv.), and anhydrous MeOH were added, and the reaction mixture was stirred for 12-14 h at rt. A saturated solution of Rochelle's salt was added and the mixture was stirred for 30 min. The solution was diluted with water and CH₂Cl₂ and the aqueous phase was extracted with CH₂Cl₂ (3x). The combined organic phases were dried over Na₂SO₄, filtered, and the solvent was removed under reduced pressure. The desired alkyne was isolated by flash chromatography on silica gel.

### C.2 Synthesis of α-Amino Alkynes from Fmoc-protected Weinreb amides

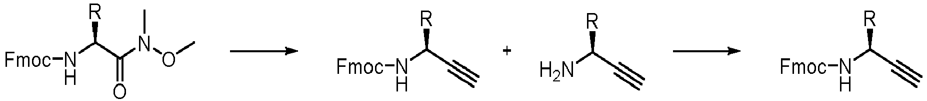

R = amino acid specific side chain

The corresponding Weinreb amide (1 equiv.) was placed in a flame dried flask under argon and dissolved in anhydrous CH₂Cl₂ (0.1 M). The solution was cooled to -78 °C (dry ice/acetone bath), and 1 M DIBAL-H in toluene was added dropwise (3 equiv.). After 1 h of stirring, the reaction was checked for completion by TLC. If the reaction was not finished, 1 M DIBAL-H in toluene (1 equiv.) was added and the reaction was stirred again for 1H at -78 °C. After consumption of the starting material, the excess hydride was quenched by slow addition of anhydrous MeOH, and the reaction was allowed to warm to 0 °C (ice/water bath). K₂CO₃ (3 equiv.), dimethyl-(1-diazo-2-oxopropyl)phosphonate (2 equiv.), and anhydrous MeOH were added, and the reaction mixture was stirred for 12-14 h at RT. A saturated solution of Rochelle's salt was added and the mixture was stirred for 30 min. The solution was diluted with water and CH₂Cl₂, and the aqueous phase was extracted with CH₂Cl₂ (3x). The combined organic phases were dried over Na₂SO₄, filtered, and the solvent was removed under reduced pressure. If cleavage of the Fmoc protecting group was detected by TLC, the crude mixture was dissolved in CH₂Cl₂ (0.1 M according to initial scale). DIPEA (2.5 equiv.) and Fmoc-OSu (2 equiv.) were added, and the reaction was stirred for 12-14 h at rt. The reaction mixture was then diluted with CH₂Cl₂ and water. The aqueous phase was extracted with CH₂Cl₂ three times. The combined organic phases were dried over Na₂SO₄, filtered, and the solvent was removed under reduced pressure. The desired alkyne was isolated by flash chromatography on silica gel.

### C.3 Synthesis of α-Amino Alkynes from Fmoc-protected α-Amino Alcohols

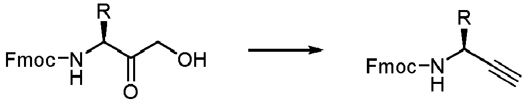

R = amino acid specific side chain

DMSO (2.2 equiv.) was dissolved in anhydrous CH₂Cl₂ (1 M) and cooled to -45 °C (dry ice/MeCN bath) under Argon. Oxalyl dichloride (1.2 equiv.) was added dropwise at -45 °C under development of gas. The solution was stirred for 5 min, before the corresponding Fmoc-protected α-amino alcohol (1 equiv., 0.13 M in CH₂Cl₂) was added dropwise at -45 °C and stirred for 30 min. DIPEA (3 equiv.) was added, the reaction was warmed to -20 °C (NaCl/ice bath) and monitored by TLC until completion. The solution was then diluted with CH₂Cl₂, and the organic layer was extracted with water, 1 M NaHSO₄, and water. The combined organic phases were dried over Na₂SO₄, filtered, and the solvent was removed under reduced pressure. The crude reaction workup was subsequently dissolved in anhydrous MeOH (0.1 M according to initial scale), K₂CO₃ (3 equiv.), and dimethyl-(1-diazo-2-oxopropyl)phosphonate (2 equiv.) were added, and the reaction mixture was stirred for 12-14 h at RT. The reaction mixture was diluted with water, and the aqueous phase was extracted with CH₂Cl₂ three times. The combined organic phases were dried over Na₂SO₄, filtered, and the solvent was removed under reduced pressure. If cleavage of the Fmoc protecting group was detected by TLC, the crude mixture was dissolved in CH₂Cl₂ (0.1 M according to initial scale). DIPEA (2.5 equiv.) and Fmoc-OSu (2 equiv.) were added, and the reaction was stirred for 12-14 h at rt. The reaction mixture was then diluted with CH₂Cl₂ and water. The aqueous phase was extracted with CH₂Cl₂ three times. The combined organic phases were dried over Na₂SO₄, filtered, and the solvent was removed under reduced pressure. The desired alkyne was isolated by flash chromatography on silica gel.

### General Procedure D for the Determination of the Optical Purity of α-Amino Alkyne Building Blocks: Synthesis of Dipeptides from α-Amino Alkynes

### D.1 Synthesis of Dipeptides from Boc-protected α-Amino Alkynes

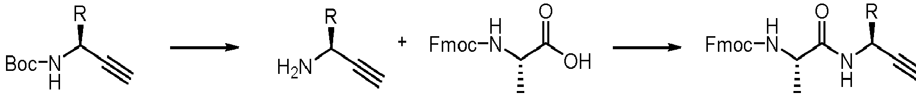

R = amino acid specific side chain

The corresponding α-amino alkyne (1 equiv.) was dissolved in a solution of CH₂Cl₂/TFA/H₂O (75:20:5) (0.05M) and the reaction was stirred for 15-30 min. After completion of the reaction, the solvent was removed under reduced pressure. Residual amounts of water and TFA were removed by coevaporation with toluene. The residue was dissolved in CH₂Cl₂ (0.1 M), and PG-Ala-OH (2 equiv., PG = Boc or Fmoc), (benzotriazol-1-yloxy)tris(dimethylamino)phosphonium hexafluorophosphate (BOP, 2 equiv.), and DIPEA (5 equiv.) were added. The reaction was stirred at rt and monitored by TLC until completion. The solvent was removed from the crude mixture under reduced pressure, and the desired dipeptide was isolated by flash chromatography on silica gel.

### D.2 Synthesis of Diastereomers from Fmoc-protected α-Amino Alkynes

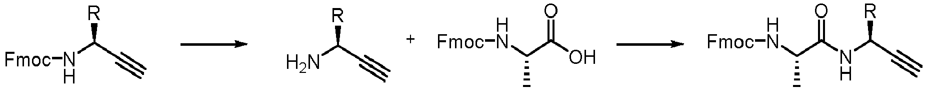

R = amino acid specific side chain

The corresponding α-amino alkyne (1 equiv.) was dissolved in 25% piperidine in DMF (0.05 M) and the reaction was stirred for 15-30 min at rt. Ice-cold H₂O was added to the reaction mixture and extracted with EtOAc (3x). The combined organic fractions were dried over MgSO₄, filtered, and the solvent was removed under reduced pressure. The residue was dissolved in CH₂Cl₂ (0.1 M), and Fmoc-Ala-OH (2 equiv.), BOP (2 equiv.), and DIPEA (5 equiv.) were added. The reaction was stirred at rt and monitored by TLC until completion. The solvent was removed from the crude mixture under reduced pressure, and the desired diastereomer was isolated by flash chromatography on silica gel.

General Procedures E: Manual Solid Phase Peptide Synthesis The rink amide MBHA LL resin (ca. 100 mg, 0.03 - 0.04 mmol) was placed in a polypropylene syringe with a polyethylene frit and a Teflon tap and swollen repeatedly in CH₂Cl₂ and DMF. 20% piperidine in DMF was used to cleave the Fmoc protecting group (3 x 3 min, rt). For elongation of the sequence, the Fmoc-protected amino acids or DOTA-tris(tert-butyl ester) (2 equiv., 0.06 mmol), HATU (1.9 equiv., 0.057 mmol) and DIPEA (5 equiv., 0.15 mmol) in DMF (total 3 mL) were added to the resin. The suspension was shaken for 1 h at rt. The solvent was removed by filtration, and the resin was repeatedly washed with DMF and CH₂Cl₂. Completion of the reaction was monitored by the Kaiser test, and the coupling was repeated if necessary.

General Procedure F: Introduction of the Azido Functionality on the N-Terminus of the Peptide on Solid Support After cleavage of the Fmoc protecting group, the free N-terminal amine was treated with imidazole-1-sulfonyl azide hydrochloride (3 equiv., 0.09 mmol), DIPEA (9 equiv., 0.27 mmol), and a catalytic amount of CuSO₄ (0.01 equiv., 0.03 µmol) in DMF (total 2 mL). The suspension was shaken for 1 h at rt. The solvent was removed by filtration, and the resin was repeatedly washed with a 0.5% solution of sodium diethyldithiocarbamate in DMF, DMF, and CH₂Cl₂. Completion of the reaction was monitored by the Kaiser test and repeated if necessary.

### General Procedure G: Solid Phase Copper(I)-Catalyzed Cycloaddition (CuAAC)

The Fmoc-protected α-amino alkyne (2 equiv., 0.06 mmol), DIPEA (1 equiv., 0.03 mmol), tetrakis(acetonitrile)copper(I) hexafluorophosphate (0.5 equiv., 0.015 mmol), and tris[(1-benzyl-1H-1,2,3-triazol-4-yl)methyl]amine (TBTA, 0.5 equiv., 0.015 mmol) in DMF (2 mL) were added to the N-terminal azide functionality, and the suspension was shaken for 12-14 h at rt. The solvent was removed by filtration, and the resin was repeatedly washed with a 0.5% solution of sodium diethyldithiocarbamate in DMF, DMF, and CH₂Cl₂. Completion of the reaction was monitored by a colorimetric test for aliphatic azides.

### General Procedure H: Solid Phase Ruthenium-Catalyzed Cycloaddition (RuAAC)

The Fmoc-protected α-amino alkyne (2 equiv., 0.06 mmol) and (chloro(pentamethylcyclopentadienyl) (cyclooctadiene)rutheniu m (CpRu(COD)Cl, 0.5 equiv.)in DMF (2 mL) were added to the N-terminal azide functionality under argon atmosphere and the suspension was shaken for 12-14 h at rt. The solvent was removed by filtration, and the resin was repeatedly washed with a 0.5% solution of sodium diethyldithiocarbamate in DMF, DMF, and CH₂Cl₂. Completion of the reaction was monitored by a colorimetric test for aliphatic azides

### General Procedure I: Cleavage and Purification of the Peptide Conjugates

After the final coupling of the N-terminal chelator DOTA-tris(tert-butyl ester), the conjugates were deprotected and cleaved from the resin using TFA/TIS/H₂O/phenol (92.5/2.5/2.5/2.5, 6 mL) with agitation for 5 h at rt. The cleavage mixture was separated from the resin by filtration and a stream of nitrogen was applied for evaporation of the volatile components. The crude peptide was then precipitated by the addition of ice-cold diethyl ether (15 mL). After centrifugation (1800 rpm, 5 min) and two washing steps with ice-cold diethyl ether, the crude peptide conjugates were dissolved in 20% CH₃CN in water (1 mg/mL) and purified by reverse phase semipreparative HPLC. Subsequent lyophilization gave the final products as white powders.

Compounds are shown in Table 1. The new compounds are TZMG 876 and NMG 86. The other compounds are known from WO 2019/057445 A1.

**Table 1: Names and sequences of mono- and multi-triazolominigastrins; Ψ[Tz] stands for a 1,4-disubstituted 1,2,3-triazole**

| Compound | Sequence | Mwt (g/mol) |
|---|---|---|
| TZMG **6** | DOTA-DGlu-Ala-Tyr-***Ψ*[Tz]**-Gly-Trp-Nle-Asp-Phe-NH₂ | 1409.5 |
| TZMG **86** | DOTA-DGlu-***Ψ*[*Tz*]**-Ala-Tyr-***Ψ*[Tz]**-Gly-Trp-Nle-Asp-Phe-NH₂ | 1433.5 |
| TZMG **876** | DOTA-DGlu-***Ψ*[Tz]**-Ala-***Ψ*[Tz]**-Tyr-***Ψ*[Tz]**-Gly-Trp-Nle-Asp-Phe-NH₂ | 1457.6 |
| NMG **6** | DOTA-(DGlu)₅-DGlu-Ala-Tyr-***Ψ*[Tz]**-Gly-Trp-Nle-Asp-Phe-NH₂ | 2055.1 |
| NMG **86** | DOTA-(DGlu)₅-DGlu-***Ψ*[Tz]**-Ala-Tyr-***Ψ*[Tz]**-Gly-Trp-Nle-Asp-Phe-NH₂ | 2079.1 |

**Table 2: Summary of synthesis scale, yields, and purities of the novel triazolominigastrins.**

| Compound | Scale (mmol) | Yield (%) | Purity (%) |
|---|---|---|---|
| TZMG **6** | 0.041 | 13.9 | > 99 |
| TZMG **86** | 0.0528 | 10.1 | 97.9 |
| TZMG **876** | 0.037 | 16.1 | > 99 |
| NMG **6** | 0.032 | 19.6 | 98.1 |
| **NMG 86** | 0.046 | 11.5 | > 99 |

### (Radio)metal Labeling

Stock solutions were prepared by dissolving the reference substances (Nle¹⁵]-MG11 and PP-F11-N) or the triazolominigastrins (1 mg, 750 nmol) in ammonium acetate buffer (50 µL, 0.5 M, pH 5.5) and addition of water to a final peptide concentration of 250 µM (approx. 0.3 mg/mL). For in vitro experiments, DOTA-functionalized compounds (1 nmol, 4 µL of 250 µM stock solution) were added to a mixture of aq. HCl (22.5 µL, 0.05 M, pH 1.3), ammonium acetate buffer (10 µL, 0.5 M, pH 5.5) and aq. sodium ascorbate (5 µL, 0.5 M). 20-25 MBq of [¹⁷⁷Lu]LuCl₃ (ca. 2.5 µL, in 0.04 M HCl, 20-25 MBq/nmol) were added and the mixtures were heated to 95 °C for 20 min in a heating block. After labeling, a 1 µL aliquot of the labeling mixture was added to aq. DTPA (200 µL, 25 µM) for quality control by γ-HPLC.

For the labeling with non-radioactive ^{nat}Lu, the test compounds (25 nmol, 100 µL, 250 µM) were mixed with a 5-molar excess of aq. [^{nat}Lu]LuCl₃ (125 nmol, 12.5 µL, 10 mM), ammonium acetate (5 µL, 0.5 M, pH 5.5) and heated to 95 °C for 20 min in a heating block.

For in vivo experiments, DOTA-functionalized compounds were labeled with higher amount of [¹⁷⁷Lu]LuCl₃ resulting in specific activities of 45-55 MBq/nmol and - after quality control - diluted with PBS to reach concentrations of 100 pmol/mL.

### Cell Culture

Human epidermoid carcinoma cells A431 stably transfected to express the CCK2R (A431-CCK2R cells)[Aloj L., Journal of Nuclear Medicine, 2004, 45, 485-494] were grown in monolayers in Nunclon™ Delta treated cell culture flasks in humidified air at 5% CO₂ and 37 °C. The cells were maintained in the culture medium DMEM (high glucose (4.5 g/L)) supplemented with 20 mM L-Gln and 10% FCS. The culture was passaged regularly at 80 to 90% confluency using a 0.25% trypsin 0.38‰ EDTA solution. Assays were conducted in the assay medium DMEM (high glucose) containing 0.1% BSA.

### Cell Internalization Experiments

On the day prior to the experiment A431-CCK2R cells were placed in six-well plates (0.85·10⁶ cells/well) in cell culture medium and incubated overnight for attachment. On the day of the experiment, the medium was removed and the cells were washed twice with 1 mL PBS. The plates were put on ice for preparation. 0.9 mL of assay medium was dispensed to all wells except the ones for nonspecific binding. 0.2 pmol of ¹⁷⁷Lu-labeled conjugates (100 µL, 2 nM in assay medium, ca. 4.2 kBq) were dispensed to all wells. For the determination of nonspecific binding, a 5000-fold excess of minigastrin (1 nmol, 100 µL, 10 µM in assay medium) was added to 0.8 mL of assay medium containing the ¹⁷⁷Lu-labeled conjugates. The plates were incubated at 37 °C in 5% CO₂ to allow binding and internalization. The process was stopped after 30, 60, 120, and 240 min by collection of the supernatant. The cells were washed twice with PBS (each 0.6 mL). The combined supernatants represent the free, unbound fraction of radioactivity. Membrane-bound activity was determined by incubating the cells with cold saline glycine buffer (0.6 mL, 0.05 M, pH 2.8) twice for 5 min at rt. The internalized fraction was isolated by two cycles of cell lysis with NaOH (each 0.6 mL, 1 M, 10 min, rt). The radioactivity of the fractions was measured by a COBRA-II gamma counter and is represented as percentage of total applied radioactivity dosage (n=3-5 in triplicates).

### Receptor Affinity - IC₅₀ Assays

On the day prior to the experiment A431-CCK2R cells were placed in six-well plates (0.85·10⁶ cells/well) in cell culture medium and incubated overnight for attachment. On the day of the experiment, the medium was removed and the cells were washed twice with 1 mL PBS. On ice, 0.8 mL of assay medium and the radiolabeled reference compound [¹⁷⁷Lu]Lu-PP-F11N (0.2 pmol, 2 nM in assay medium, 100 µL, ca. 4.2 kBq) were dispensed to each well (final concentration in well = 0.2 nM). ^{nat}Lu-labeled test compounds were added to reach final well concentrations of 10⁻¹¹ to 5·10⁻⁶ M (100 µL of dilution series from 10⁻¹⁰ to 5·10⁻⁵ M in assay medium). Total binding of 177Lu-DOTA-PP-F11N was determined by incubation of the cells without addition of test compounds. After incubation at 4 °C for 1 h, the supernatant was removed, and cells were washed twice with 1 mL cold PBS. NaOH was added twice to all wells for cell lysis (0.6 mL, 1 M, 10 min, rt). The radioactivity associated with the lyzed cells was determined by a COBRA-II gamma counter. 50% inhibitory concentrations (IC₅₀) were calculated by normalized nonlinear regression with GraphPad Prism (n=3 in triplicates).

### Blood Plasma Stability

The ¹⁷⁷Lu-labeled compounds were diluted with 0.9% NaCl to a concentration of 3.75 µM and incubated (375 pmol, 100 µL, 7.5-12 MBq) in human blood plasma (1.5 mL) at 37 °C. At different time points (0.5, 1, 2, 4, 6 and 24 h) aliquots (75 µL) were taken and the proteins were precipitated in CH₃CN (100 µL) and centrifuged (2 min, 14680 rpm, rt). The supernatant (75 µL) was diluted with water (75 µL) and analyzed by γ-HPLC. One phase decay nonlinear regression (A = A₀ ^{∗} e^{-kt}) was used to calculate the half-lifes (t_{1/2}) of the peptide conjugates with GraphPad Prism (n=2-3).

### LogD Determination

The lipophilicity of the radiolabeled triazolopeptidomimetics (logD) was determined by the "shake flask method". The radiolabeled conjugates (10 pmol, 10 µL, 1 µM in PBS, ca. 0.25 MBq) were added to a saturated 1:1 mixture of n-octanol/PBS (1 mL, pH 7.4) and shaken vigorously by vortex for 1 min. After centrifugation (3000 rpm, 10 min), 100 µL aliquots of both phases were taken, and the radioactivity was measured in a gamma counter (n=2 in quadruplicates).

Biodistribution studies in xenografted mice All procedures were approved by the regional animal committee and were in accordance with international guidelines on the ethical use of animals. Six-week old female CD1 nu/nu mice (Charles River Laboratory, Germany) were inoculated with 5·10⁶ A431-CCK2R cells. They were grown for 2 weeks until they had reached a size of 50-200 mm³. On the day of the experiment, they were injected with 10 pmol of the respective ¹⁷⁷Lu-labeled compound in 100 µl PBS (ca. 0.5 MBq) via the tail vein. The mice were sacrificed by CO₂ suffocation 1, 4 or 24 hours p.i., and the organs (blood, heart, lungs, spleen, kidneys, pancreas, stomach, intestines, liver, muscle, bone, tumor) were harvested by dissection, weighed and measured in a gamma counter (n=4 per time point). The content of stomach and intestines was removed prior to the analysis. For blocking experiments, the mice were injected with 100 mg of minigastrin (ca. 60'000 pmol, 6000 fold excess) in 100 µl PBS prior to the injection of the radiolabeled compounds (n=4). Tissue distribution data were calculated as percent injected activity per gram of tissue (% ID/g), and statistical analysis was performed using GraphPad Prism.

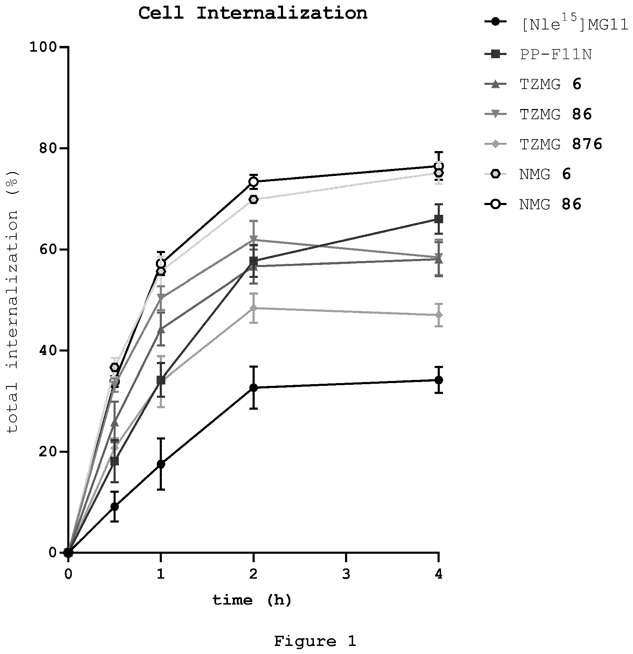

Figure 1 illustrates the specific internalization for the the ¹⁷⁷Lu-labeled minigastrin derivates [Nle¹⁵]-MG11 and PP-F11N as a reference and the derivated minigastrins having incorporated Tz (1,4-disubstituted 1,2,3-triazole) at different positions in the N-terminal region of the sequence. TZMGs 6, 86, and 876 have faster cell internalization kinetics and reach higher cell internalization after 4 h than [Nle¹⁵]MG11. NMGs 6 and 86 have faster cell internalization kinetics and reach higher cell internalization after 4 h than PP-F11N.

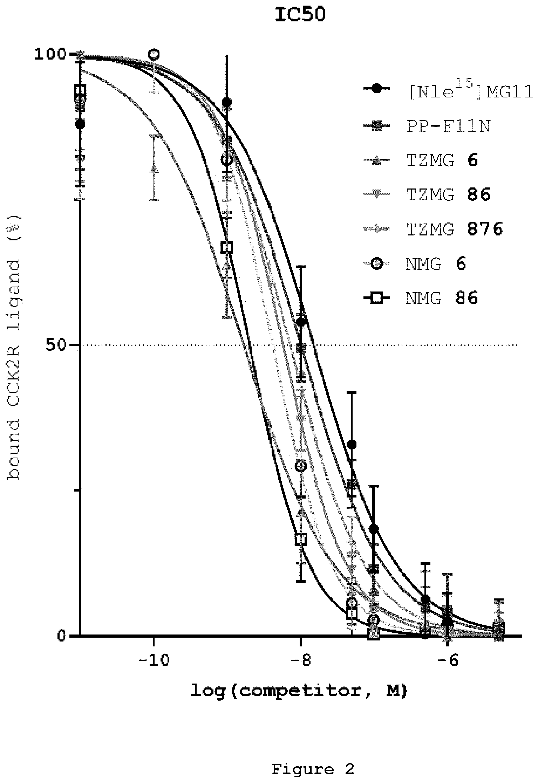

Figure 2 illustrates the half maximal inhibitory concentration IC₅₀ for the ^{nat}Lu-labeled minigastrin derivates also present in Figure 1. Important here is that the IC₅₀ values for all TZMGs and NMGs are lower than for [Nle¹⁵]-MG11 and PP-F11N.

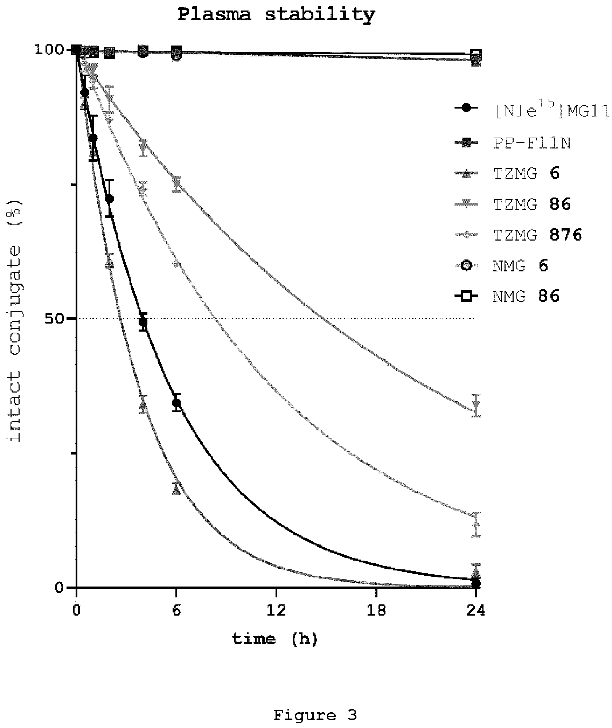

For these minigastrin derivates, Figure 3 illustrates the plasma stability as compared to the ¹⁷⁷Lu-labeled reference minigastrin derivates [Nle¹⁵]-MG11 and PP-F11N. The single amide-to-triazole substitution TZMG 6 leads to reduced stability in plasma. The multi-TZMGs 86 and 876 shown significantly higher stability than [Nle¹⁵]-MG11. All compounds bearing the N-terminal hexaglutamate motif (PP-F11N, NMG 6, NMG 86) show a high stability in plasma over 24 hours.

## Claims

**1.** A minigastrin derivate, in particular mono- and multi-triazolominigatrins for targeting of CCK2R-positive neoplasms, having the formula:
X-Z-Ψ[Tz]-Ala-Ψ[Tz]-Tyr-Ψ[Tz]-Gly-Trp-Nle-Asp-Phe-NH₂ (Y),
or
X-Z-DGlu-Ψ[Tz]-Ala-Tyr-Ψ[Tz]-Gly-Trp-Nle-Asp-Phe-NH₂ (Y),
or
X-Z-DGlu-Ala-Tyr-Ψ[Tz]-Gly-Trp-Nle-Asp-Phe-NH₂ (Y),
while Ψ[Tz] stands for a 1,4-disubstituted or a 1,5-disubstituted 1,2,3-triazole, X stands for a chemical group attached to the peptide for the purpose of diagnostic and/or therapeutic intervention at CCK2R-relevant diseases, Y stands for C-terminal modifications of the peptide, such as amide, primary and secondary amides, free carboxylic acids and carboxylic ester derivatives including but not limited to amides and esters derived from linear or branched alkyl-,alkenyl-, alkynyl- aromatic-, and heterocyclic alcohols, and Z stands for a linker or DGlu* wherein DGlu* stands for a chain of DGlu having 1 to 6 repetitions (-DGlu- to -DGlu-DGlu-DGlu-DGlu-DGlu-DGlu-).

**2.** The minigastrin derivate according to claim 1, wherein X stands for a radionuclide including a chelator for radiometals.

**3.** The minigastrin derivate according to claim 2,
wherein the chelator for radiometals is selected from a group consisting of DOTA (1,4,7,10-tetraazacyclododecane-1,4,7,10-tetraacetic acid), NOTA, NOTAGA, CHX-A"-DTPA and TCMC.

**4.** The minigastrin derivate according to claim 2 or 3, wherein the radionuclide is selected from the group consisting of ¹⁷⁷Lu, ⁹⁰Y, ¹¹¹In, ⁶⁷Ga/⁶⁸Ga, ^{99m}Tc, ⁶⁴Cu/⁶⁷Cu, ²²⁵Ac, ²¹³Bi, ²¹²Pb, and ²²⁷Th.

**5.** The minigastrin derivate according to any of the preceding claims, wherein the minigastrin derivate having the formula:
DOTA-DGlu-Ψ[Tz]-Ala-Ψ[Tz]-Tyr-Ψ[Tz]-Gly-Trp-Nle-Asp-Phe-NH₂ or
DOTA-(DGlu)₅-DGlu-Ψ[Tz]-Ala-Tyr-Ψ[Tz]-Gly-Trp-Nle-Asp-Phe-NH₂.

**6.** The minigastrin derivate according to claim 5, wherein DOTA-DGlu-Ψ[Tz]-Ala-Ψ[Tz]-Tyr-Ψ[Tz]-Gly-Trp-Nle-Asp-Phe-NH₂ is labeled with ¹⁷⁷Lu or DOTA-(DGlu)₅-DGlu-Ψ[Tz]-Ala-Tyr-Ψ[Tz]-Gly-Trp-Nle-Asp-Phe-NH₂ is labeled with ¹⁷⁷Lu.

**7.** The minigastrin derivate according to claim 1, wherein X stands for an optically active chemical compound.

**9.** The minigastrin derivate according to claim 1, wherein X stands for a chemotherapeutic active compound or any other therapeutic active compound like tyrosine kinase inhibitor or immunogenic active compound.

**10.** The minigastrin derivate according to claim 1, wherein X stands for a nanoparticle or a liposome which have a diagnostic function (e.g. optical active or MRI contrast agent) or which have therapeutic function by themselves or are loaded with an active compound.
